# EUROPEAN PATENT APPLICATION

(11) **EP 2 716 768 A1**
(43) Date of publication of application: **09.04.2014**
(21) Application number: 13160956.2
(22) Date of filing: 25.03.2013
(51) Int. Cl.: C12Q 1/68

(54) **Cell-based assay for assessing TNF alpha inhibitors**

(30) Priority: 04.10.2012 EP 12006880
(71) Applicant: IN. Medica, d.o.o., 8310 Sentjernej (SI)
(72) Inventor: Barlic, Ariana, 1000 Ljubljana (SI); Kregar-Velikonja, Nevenka, 8351 Straza (SI)
(74) Representative: Hoefer & Partner

(57) **Abstract**

A chondrocyte cell model for evaluation of the functional effect of medicines (biopharmaceuticals, small molecules) on cartilage tissue. An in vitro method for assessing TNFα inhibitory activity of a test compound comprising contacting a chondrocyte cell culture with TNFα in presence and absence of the test compound and determining the expression levels of at least 6 marker genes selected from a group comprising at least IL-1RA, IL-6, IL-8, IL-32, MMP1, MMP3, MMP13, MCP-1, TLR2 and VCAM-1. Identified compounds may be useful for treating or preventing diseases associated with elevated TNFα levels, such as inflammatory diseases including rheumatoid arthritis and osteoarthritis.

## Description

### Field of the invention

The present invention is concerned with a cell model for identification, characterisation, and/or evaluation of the functional effect of a compound, and the use of said compound as an agent to prevent or treat inflammatory diseases.

### Background

Functional assays serve multiple purposes in the characterization of protein or nucleic acid products. These tests act to complement physicochemical analyses and are a quality measure of the function of the protein product.

Over the next few years it is anticipated that there is going to be an explosion in the numbers of biosimilar products, in particular pharmaceutical products coming to market. Consequently, there will be a proportional need to provide full characterization of such biopharmaceuticals. According to FDA guidance for demonstrating biosimilarity to a reference product, manufacturers should consider in a stepwise approach a number of factors including expression system, manufacturing process, assessment of physiochemical properties, functional activities, etc. (1).

FDA guidance suggests also that »if the clinically relevant mechanism(s) of action are known for the reference product, one or more of the functional assays should reflect these mechanisms of action to the extent possible. The assessment of functional activity is also useful in providing an estimate of specific activity of the product, as an indicator of manufacturing process consistency, as well as product purity and stability (2).

Biopharmaceuticals have proven utility in the field of inflammatory diseases. One example for inflammatory diseases is Rheumatoid Arthritis (RA), which is a chronic inflammatory disease affecting the synovial tissue in multiple joints. RA is characterized by an influx of inflammatory cells, which leads to hyperplasia and eventually destruction of cartilage and bone (3). The introduction of biological agents has dramatically altered the therapy for patients with RA. Well established currently available biological agents include tumor necrosis factor alpha (TNFα) inhibitors (infliximab, etanercept, adalimumab, certolizumab pegol and golimumab), an interleukin-1 (IL-1) receptor antagonist (anakinra), a B-cell-depleting agent (rituximab) and an inhibitor of T-cell costimulation (abatacept). TNF inhibitors were the first biologics to be added to the therapeutic arsenal for RA treatment (4).

The treatment of RA is difficult as this disease does respond mostly on medications that carry a high risk of adverse side effects. In the past two principal approaches for treatment have been made: symptomatic treatment with non-steroidal anti-inflammatory drugs (NSAIDs) and disease modifying anti-rheumatic drugs (DMARDs). NSAIDs, i.e. mainly anti-inflammatory and analgetic drugs, were used for treatment or at least pain release. They only interfere with a small segment of the inflammatory cascade, namely prostaglandin generation by cyclooxygenases (COXs), but do not interfere with underlying inflammatory events or retard joint destruction.

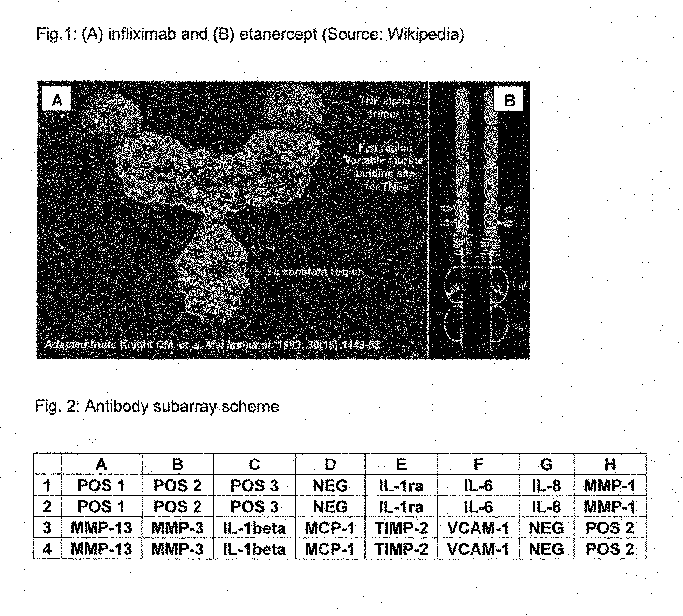

By contrast, DMARDs modify the disease process. Recently, within the group of DMARDs a new class of biological drugs has been developed based on a new understanding of the role of cytokines, particularly TNFα and IL-1, in the inflammatory process. Examples of DMARDs are methotrexate, leflunomide, or infliximab, i.e. anti-TNFα antibodies. Infliximab is a monoclonal antibody, and etanercept a fusion protein composed of TNF receptor 2 and IgG1 (Figure 1).

Some drugs have been approved like anti-TNFα antibodies. However, it was found that again the side effects of some TNFα- or IL-1 receptor antagonists are so severe that their use is restricted, although these drugs have been shown to interfere in the inflammatory process and can control the process.

All treatments proposed until today have the disadvantage of severe side effects. Therefore, it is still an objective to develop new drugs for the treatment of diseases where TNFα is involved, like arthritic diseases, e.g. rheumatoid arthritis (RA) and osteoarthritis (OA). These drugs should be as effective as NSAIDs or DMARDs but should have fewer side effects. Therefore, rapid, cost-effective, and reliable characterization and evaluation of new biopharmaceuticals, as for example TNFα inhibitors, is of the utmost importance.

Inflammatory cytokines produced by activated cells in the arthritic joints drive cartilage and bone destruction (5). Thus, cartilage is a primary target tissue in the affected joints. It was one object to find a suitable cell model for study and evaluation.

According to recently published results of new anti-TNFα biologics characterization, the neutralization of soluble TNFα in a cell-based assay is assessed predominantly by evaluating cytotoxicity in murine fibroblast L929 or other cell lines (6, 7, 8, 9).

It was the object of the present invention to provide methods to screen and analyse agents, in particular pharmaceutical agents, for their activity, to test biosimilarity with known products, to be able to compare agents in a reliable and efficient manner, and to provide methods that can be standardized. It was a further object of the present invention to provide methods for identifying, comparing and/or evaluating biopharmaceuticals and their effect on cells. In particular, it was an object of the present invention to provide means for determining biosimilarity of drugs for treating diseases where TNFα is involved, like inflammatory diseases of cartilage, bone and joints, in particular arthritic diseases, e.g. rheumatoid arthritis (RA), osteoarthritis (OA), and other types of rheumatoid conditions. Examples for such drugs are anti-TNFα biologics. Furthermore, it is an object to provide methods to screen and analyse agents that can be used in the treatment of cartilage and/or bone destroying diseases and diseases affecting joints and synovial tissue in joints.

The present invention solves these objects by providing a cell-based assay for assessing bioactivity of anti-TNFα biologics. This test is useful for identifying acitve agents and for screening and determining biosimilarity and is a tool for identification, evaluation and comparison of active agents that modulate TNFα. The method is of particular value in the early phases of drug development.

The inventors found that chondrocytes, the only cellular component in the cartilage, represent a suitable cell model for studying the neutralization of the soluble TNFα by TNFα inhibitors. Additional value of a chondrocyte cell model represents human primary cell cultures. Lately, it is very well accepted that results obtained by using primary cells instead of cell lines, provide much more physiologically relevant data. The inventors identified that neutralization of TNFα by anti-TNFα biologics is reflected as suppression of TNFα-induced gene expression. Moreover, a method for identifiying marker genes was found and 10 marker genes were selected via the application of qPCR array, which are suitable for following biological activity of anti-TNFα biologics.

The inventors furthermore found that chondrocyte's response upon TNFα stimulation can be used for detection of changes in inflammatory processes and for matrix remodeling gene expressions. The functional assay of the present invention uses a cell-model based on chondrocytes instead of mouse fibroblasts thereby yielding results of the assay that are more reliable and applicable to the real life situation occurring in the patient afflicted with a disease associated with elevated TNFα levels, such as RA or OA, because chondrocytes in the patients are cells where the TNFα levels are elevated. It was found that assessing dose-response relationship of anti-TNFα biologics by qPCR gene expression did not give applicable results as IC₅₀ could not be determined.

Compounds identified, assessed, characterized, and/or evaluated by the methods of this invention can be used as agents to prevent or treat inflammatory diseases. The cell-based assay of the present invention can be used for assessing biosimilarity of a proposed therapeutic protein product to a known reference product, as biosimilarity needs to be demonstrated based on data directly comparing the proposed protein product with the reference product. It is further intended to use the cell-based assay of the present invention as a tool to demonstrate that a proposed therapeutic protein product is biosimilar to a reference product already approved for therapeutic use under the regulations for therapeutic goods as set out by governmental agencies such as the EMA or the FDA. Biosimilarity needs to be demonstrated based on data directly comparing the proposed protein product with the reference product.

Furthermore, it was established that the use of primary chondrocytes provides an added value to this cell-based assay; intra- and interindividual variability of donors is reflected in different activity of the tested anti-TNFα biologics.

Data obtained by gene expression analysis was confirmed by antibody-based arrays for at least 6 marker genes.

The chondrocyte cell-based assay of the present invention due to the selection of at least 6 marker genes and reproducibility of the results is suitable for assessing new and known anti-TNFα biologics.

### Definitions

In this description and the claims, reference will be made to a number of terms which shall be defined to have the following meanings:

The term "inhibiting TNFα" when used in this application shall refer to inhibiting the activity or function of this cytokine, particularly to inhibiting the binding of TNFα to its receptor and inhibiting the signaling caused by that binding. The term inhibiting includes blocking, antagonizing and reducing the activity or function of TNF-α. The degree of inhibition can be determined by comparison to a reference, standard, or norm.

The term "TNFα inhibitor" when used in this application refers to any substance that inhibits, blocks, or reduces binding of TNFα to its receptor and thereby interrupts the signaling cascade. A TNFα inhibitor can also be any substance that neutralizes or antagonizes the bioactivity of TNFα; it can also be a substance that prevents release of TNFα. Preferably, a TNFα inhibitor is a substance blocking TNFα or a substance that selectively reduces TNFα release.

"Increased TNFα inhibitory activity" when used in this application refers to the situation, wherein the expression levels of assessed/measured marker genes of a test compound are lower than or equal to the corresponding expression levels of the marker genes of the known compound (reference compound). The comparison of the expression levels is based on the mean values of the measured expression levels, wherein the mean value of the expression level of the marker gene of the test compound in more than half of the measured/assessed expression levels of marker genes (e.g. at least 4/6, 5/8 or 6/10 expression levels of marker genes) is less than half the mean value compared with the expression level of the corresponding marker gene of the known reference compound. Less than half the mean value compared with the expression level of the corresponding marker gene of the known reference compound in a radar graph will mean outside the two-fold change area and closer to graph centre.

"Decreased TNFα inhibitory activity" when used in this application refers to the situation, wherein the expression levels of assessed/measured marker genes of a test compound are higher than or equal to the corresponding expression levels of the marker genes of the known compound (reference compound). The comparison of the expression levels is based on the mean values of the measured expression levels, wherein the mean value of the expression level of the marker gene of the test compound in more than half of the measured/assessed expression levels of marker genes (e.g. at least 4/6, 5/8 or 6/10 expression levels of marker genes) is more than twice the mean value compared with the expression level of the corresponding marker gene of the known reference compound. More than twice the mean value compared with the expression level of the corresponding marker gene of the known reference compound in a radar graph wll mean outside the two-fold change area and more removed from the graph centre.

"Similar TNFα inhibitory activity" when used in this application refers to the situation, wherein the expression levels of more than half of the assessed/measured marker genes of a test compound are less than twice the mean value but more than half the mean value of the corresponding expression levels of the marker genes of the known compound (reference compound). The comparison of the expression levels is based on the mean values of the measured expression levels, wherein the mean value of the expression level of the marker gene of the test compound in more than half of the measured/assessed expression levels of marker genes (e.g. at least 4/6, 5/8 or 6/10 expression levels of marker genes) is less than twice but more than half the mean value compared with the expression level of the corresponding marker gene of the known reference compound. Less than twice but more than half the mean value compared with the expression level of the corresponding marker gene of the known reference compound in a radar graph will mean within the two-fold change area of the known compound.

"Incomparable TNFα inhibitory activity" when used in this application refers to the situation, wherein the expression levels of more than half of the assessed/measured marker genes of a test compound are more than twice the mean value or less than half the mean value of the corresponding expression levels of the marker genes of the known compound (reference compound). The comparison of the expression levels is based on the mean values of the measured expression levels, wherein the mean value of the expression level of the marker gene of the test compound in more than half of the measured/assessed expression levels of marker genes (e.g. at least 4/6, 5/8 or 6/10 expression levels of marker genes) is for some more than twice and for some less than half the mean value compared with the expression level of the corresponding marker gene of the known reference compound. More than twice or less than half the mean value compared with the expression level of the corresponding marker gene of the known reference compound in a radar graph will mean outside the two-fold change area of the known reference compound, but on both sides of two-fold change.

"Biosimilar" or "biosimilarity" means that the biological product is highly similar to the reference product notwithstanding minor differences in clinically inactive components, and that there are no clinically meaningful differences between the biological product and the reference product in terms of the safety, purity, and potency of the product.

A TNFα inhibitor can be selected from an antibody that specifically inhibits TNFα or specifically blocks the binding of TNFα to its receptor, or a derivative or a fragment thereof, or an agent that specifically inhibits TNFα or specifically blocks the binding of TNFα to its receptor or specifically blocks the production of TNFα, for example a ligand that binds to TNFα or its receptor, a polypeptide or a peptide mimetic that binds to TNFα or its receptor, an aptamer or Spiegelmer that binds to TNFα or its receptor, DNA or RNA molecules that code for a peptide or polypeptide that has binding activity for TNFα or its receptor or can modulate this binding, or soluble constructs that comprise parts of the TNFα receptor and a fragment of IgG.

The term "antibody", whenever used in this application, shall comprise monoclonal antibodies and polyclonal antibodies as well as modified antibodies, like genetically engineered antibodies, chimeric antibodies, single chain antibodies or humanized antibodies. Moreover, the term "antibody" when used in this application shall also include fragments, variants and multimers.

The term "fragment of an antibody" comprises any type of fragment that is well-known in the art, like inter alia F_{ab}-fragments or F(ab')₂ fragments, and has binding affinity for TNFα or its receptor. A fragment can be derived from a polyclonal or monoclonal antibody. Methods for providing antibodies or antibody fragments are standard methods and are well known to the skilled person.

The term "ligand" as used herein relates to any compound having the ability to specifically interact such that TNFα is blocked or inactivated. The interaction can be binding to TNFα or the TNFα receptor, whereby TNFα is blocked or inactivated or can be binding to a TNFα activating substance.

The terms "peptide" or "polypeptide" respectively refer to a sequence of amino acids that can be obtained by chemical synthesis or by expression from a nucleic acid encoding the peptide or polypeptide or a combination thereof. The peptide or polypeptide can comprise naturally occurring amino acids, non-naturally occurring amino acids, modified amino acids or a combination thereof. Moreover, the peptide or polypeptide can have functional groups to introduce a function that is useful for binding to TNFα or its receptor, for immobilizing a complex of the polypeptide or peptide with TNFα, or for modulation of the TNFα signaling.

The term "nucleic acid encoding a peptide or polypeptide that inhibits or blocks or modulates TNFα" refers to a nucleic acid that can be selected from any biological or synthetic source or may be contained in nucleic acid libraries or databases. It includes DNA and RNA, and it includes nucleic acids that comprise modified nucleotides. The nucleic acid can be any type of nucleotide molecule like circular or linear and/or single-, double-stranded or partially double-stranded nucleic acid. Generally cytokines play a role in inflammatory diseases. Irregular and/or abnormal inflammation is a major component of a wide range of human diseases, for example in rheumatoid diseases, like rheumatoid arthritis and osteoarthritis.

"Osteoarthritis" (OA), also referred to as degenerative joint disease or wear-and-tear arthritis, is caused by the breakdown of joint cartilage. Cartilage acts as a cushion between the bones that form a joint. Cartilage loss can cause bone to rub on bone in a joint, a condition that is very painful. Usually osteoarthritis begins in a single joint.

"Rheumatoid arthritis" (RA) is a chronic, inflammatory and destructive type of arthritis. It is classified as an autoimmune disease. It is primarily the synovium that is affected by rheumatoid arthritis, but organs body-wide can be affected as well. Multiple joints are usually involved with rheumatoid arthritis, often six or more joints, although in the early stages of the disease, only one or a few joints might be afflicted.

RA affects 0.5 to 1% of the population in the industrialized world and commonly leads to significant disability and a consequent reduction in quality of life. It is two to three times more frequent in women than in men and can start at any age, with a peak incidence between the fourth and sixth decade of life. Rheumatoid arthritis is associated with high costs and, if not treated appropriately, with a reduction in life expectancy.

Therefore, the evaluation of compounds or agents that modulate the activity of cytokines, in particular of TNFα is of high importance as they can play a role in the treatment of these chronic diseases.

### Description of drawings

The invention is also explained with reference to the following figures.
Figure 1 shows the structure of infliximab and etanercept.
Figure 2 shows the subarray scheme used in the antibody-based array.
Figure 3 shows the experimental setup.
Figure 4 shows cell morphology after 24 hours stimulation with TNFα.
Figure 5 shows response of different biological samples upon TNFα stimulation and neutralization upon anti-TNFα biologics treatment obtained by qPCR array experiments. Log₂ relative quantities (Ln(RQ)) versus particular gene expression are presented. Gene numbering is presented in Table 1. Higher column means higher gene expression upon TNFα stimulation. Calibrator is a non-treated sample.
Figure 6 shows dose-response assessment of infliximab and etanercept in varying concentrations. Gene expressions are normalized to TNFα stimulated samples. Examples of 3 genes are shown.
Figure 7 shows qPCR array data of sample 1 treated with TNFα and anti-TNFα at two time-points. Ln relative quantity vs. gene expression is presented. Expression profiles between the two time-points should be compared to assess assay reproducibility.
Figure 8 shows qPCR array data of sample 2 treated with TNFα and anti-TNFα at two time-points. Ln relative quantity vs. gene expression is presented. Expression profiles between the two time-points should be compared to assess assay reproducibility.
Figure 9 shows qPCR array data of sample 3 treated with TNFα and anti-TNFα at two time-points. Ln relative quantity vs. gene expression is presented. Expression profiles between the two time-points should be compared to assess assay reproducibility.
Figure 10 shows protein expression of 3 biological samples assessed by Antibody array. Log₂ signal intensity values are presented. Data is normalized to non-treated samples.

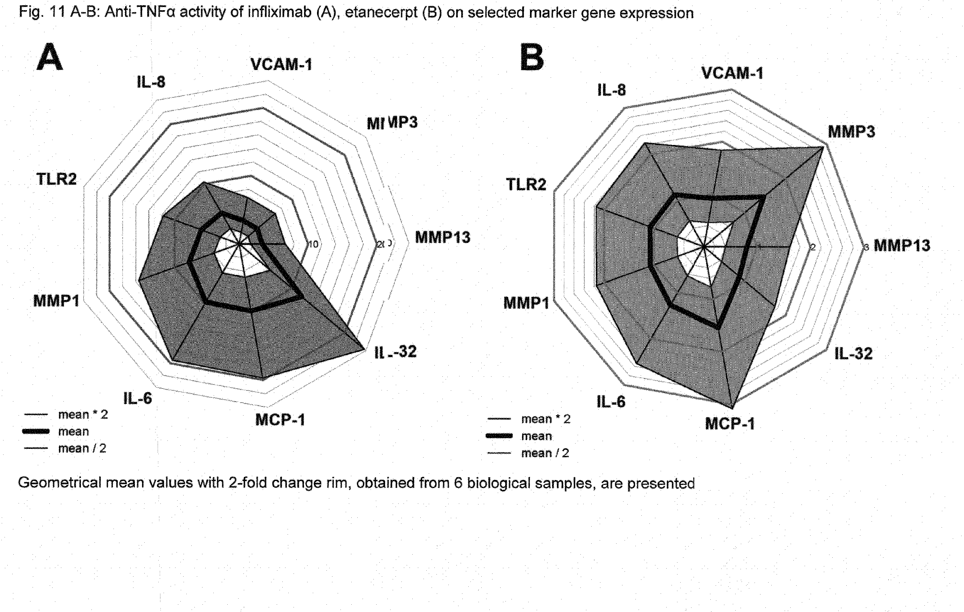

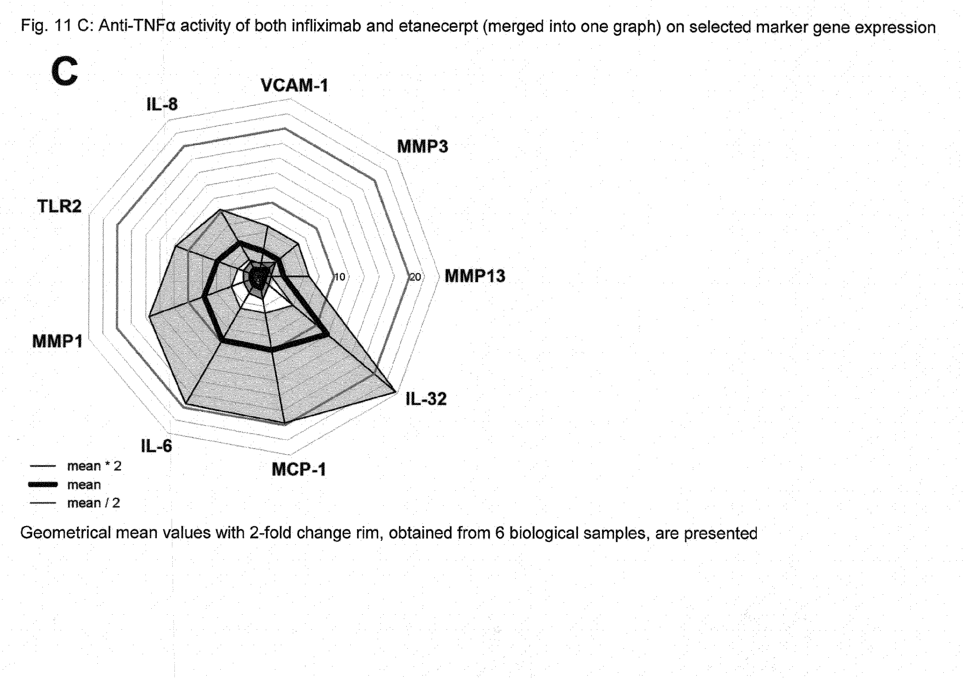

Figure 11 shows anti-TNFα activity of infliximab and etanercept on selected marker gene expression; Geometrical mean values with 2-fold change rim, obtained from 6 biological samples, are presented on radar graphs. A: infliximab, B etanercept, C merged graphs of both infliximab and etanercept

### Detailed Description of the invention

The present invention provides a cell-based assay which can be used for screening, evaluation and comparison of TNF-α inhibitory activity of test compounds. It was found that by evaluation of the expression of marker genes in TNF-α stimulated cells the inhibition of TNF-α can be determined and monitored.

A chondrocyte cell model is used for the evaluation and comparison of TNF-α inhibitory activity of test compounds. It has been found that chondrocytes are particularly useful for analysing the functional effect of medicines, for example biopharmaceuticals and small molecules, on cartilage tissue.

For this purpose the present invention provides marker genes which are effective for the assessment of TNF-α inhibitory activity. A method for determining these marker genes is described in the examples. Further marker genes can be identified by using these methods and a marker gene is useful if it fulfills the criterion that ΔΔCt ≥ 3, in a test as described in example 5.

It has been found that at least 6 marker genes have to be used to yield reliable results. These marker genes can be selected from a group consisting of interleukins, namely IL-1RA, IL-6, IL-8, IL-32, matrix metalloproteinases, namely MMP-1, MMP-3, MMP-13, monocyte chemotactic protein-1 (MCP-1), toll-like receptor 2 (TLR-2), and vascular cell adhesion molecule 1 (VCAM-1). The assay yields reliable results if at least 6 out of this group of markers is used. In a preferred embodiment the set of marker genes comprises at least 8 of these marker genes. In another embodiment all of these marker genes are used for the validation.

Furthermore in a preferred embodiment IL-6, IL-8, IL-32, MMP-1, MMP-3, MMP-13, TLR-2, and VCAM-1 are used for the validation.

In one embodiment the set of marker genes comprises at least IL-6, IL-8, MMP-1, MMP-3, MMP-13, and VCAM-1 out of the group of marker genes.

The TNF-α inhibitory activity of a test compound is assessed by comparing the expression levels of marker genes in the presence of a test compound, and in the absence of a test compound and in the presence of a known compound. For this assessment in a TNF-α stimulated chondrocyte cell culture the expression levels are determined in three different runs, first in the absence of any test or known compound, second in the absence of any test compound but in presence of a known compound for which TNF-α inhibitory activity is known, and third in the presence of the test compound but in the absence of the known compound.

Test and reference compounds can be used at various concentrations but for comparison should be used in one test set at about the same concentration or in about substantially the same amount. If different concentrations are used the amounts can be normalized as is known in the art. The skilled person can determine the optimal concentration for a test set by routine tests.

The chondrocyte cell culture is stimulated with TNF-α, before the compounds are added or together with and in the presence of anti-TNF-α agents to be tested and the effect is measured thereafter. The contacting and stimulation step is done as known to the skilled person.

In the examples it is shown how the TNF-α concentration can be optimized. The optimal amount of TNF-α that is used to stimulate the cells depends from the cell culture and the conditions and can be determined by the skilled artisan with the teaching found in this description and in the examples. It has been found that a concentration in the range of about 0.05 to 15 ng/mL is suitable, with a range of about 0.1 to 10 ng/mL being preferred. More preferable the concentration of TNF-α is in a range between 0.5 and 5 ng/mL.

Embodiments of the contacting step are also described in the examples. This contacting is done as known in the art.

After the contact of the TNF-α stimulated chondrocytes with either the test compound or a known compound the expression levels of marker genes are measured. The expression levels are determined as is well-known in the art and is described in the examples. A suitable method is real time quantitative PCR (qPCR) using markers, like fluorescent or radio active markers. The tests are preferably performed in duplicate or triplicate. A housekeeping gene like GUS can be used for the quantification. The Cq values can be determined as the cycle number at which the fluorescence intensity reaches a threshold, like 0.1.

Methods, detection systems and evaluation software are known and commercially available. Examples are ABI 7900HT Sequence Detection System, or *7900HT* Fast Real-Time PCR System (Applied Biosystems); TaqMan® gene expression master mix and TaqMan® gene expression assays (Applied Biosystems); and ABI 7900HT Sequence Detection System version 2.4 and RQ Manager software (Applied Biosystems).

Relative expression values indicate if the gene of interest is expressed to a greater or lower extent than the endogenous control gene. Normalized expression values are reported in arbitrary units and can only be used for comparative analysis among samples. For qPCR array data analysis, 2-^{ΔΔCt} formula can be applied.

The level of marker genes is also measured in a control experiment with no test compound which is used as calibrator. Furthermore, another control can be used by measuring the expression level in chondrocytes that have neither been stimulated with TNF-α nor been contacted with a TNF-α inhibitory compound.

The expression levels obtained in the presence of a known compound having TNF-α inhibitory activity can be compared to the expression levels obtained with a test compound. The known compound can be any compound for which it is known that it has TNF-α inhibitory activity. If the test is used to determine biosimilarity, the known compound is that compound to which biosimilarity shall be determined.

Test compounds can be either compounds that are screened for TNF-α inhibitory activity or compounds the TNF-α inhibitory activity of which shall be compared with a known compound. This can be done to select compounds having a potential as TNF-α inhibitory compound. Moreover, the test compound can also be a compound for which is known that it has TNF-α inhibitory activity and for which biosimilarity with a known compound shall be proven.

The test can be performed in one or more biological samples. In case an unknown compound is tested two or more biological samples, which can be the same or different, can be used in parallel for testing an unknown compound, i.e. test compound, and a reference or control compound. If more than one biological sample is used, geometrical mean values, being calculated from averaged expression values, can be presented on the radar graph, where test and control compound are plotted. In case only one biological sample is used averaged expression values are plotted.

In one embodiment 6 biological samples can be used for testing an unknown compound and the geometrical mean is used for the radar graph.

The expression levels determined as above are used for evaluation. In a preferred embodiment, the tests are performed at least in duplicate or in triplicate; in this case the mean values are used for comparison of the expression levels.

For the comparison of expression levels, in a preferred embodiment the Cq value of the housekeeping gene is subtracted from the Cq values of the target sequences, to define the activity of a test compound compared to the activity of a reference compound, the marker set as defined above is used and the expression levels are determined. A statistical model has been developed to allow easy comparison. The statistical model is also referred to as radar graph in the following. A graph is established, where at the same time the expression levels of all marker genes of the marker set are presented as lines of the average values (or geometrical mean obtained therefrom) of data normalized to the non-treated sample. Value 1 in the center of the graph means total inhibition. The expression levels obtained by test compound treatment are positioned into the graph. If the expression levels of the test compound treatment are positioned in the outer rim compared to a reference compound it means that the inhibition activity is weaker. If the expression level of a test compound compared to the expression level of a reference compound is in the inner rim it means that the inhibition activity is stronger. If both the expression level of the test compound and of the reference compound are within the rim, both have a similar activity. The two rims, the outer rim and the inner rim define a two-fold change area which is used to define similarity or non-similarity. The outer rim is defined by a two-fold cut-off, whereas the inner rim is defined by a half-fold cut-off. Each value obtained for the expression of a marker gene is filled in the radar graph and checked if it is outside the two-fold change area or inside. Then it is determined, how much markers of the marker set are within the two-fold change area and how many are beyond the outer rim or beyond the inner rim. Based on the position of the values, the activity of the test compound can be assessed.

The comparison, thus, is facilitated by using a so-called radar chart or radar graph, wherein as many axis are provided as markers, wherein each axis starts in the center of the chart and ends on an outer ring, and wherein for each marker the values are plotted along the axis. After having plotted the values for the reference compound, one rim is drawn along the two-fold values, which is called outer rim, and one rim is drawn for the half-fold values, which is called inner rim. The area spanned by the two rims is called "two-fold change area". The evaluation then can be described as follows:

For evaluation the mean values for the expression level of a set of marker genes are compared with the mean value for the reference compound. The following situations can occur:
1. The expression level for all tested marker genes for a test compound is lower or equal than for the reference compound and more than half of the measured expression levels, such as four out of six, are lower or equal than the expression levels for the reference compound and the mean value of a tested compound is outside the two-fold change area in more than half of the tested marker genes. In other words, the mean value for the gene expression is outside the two-fold change area and closer to the graph center in more than half of the tested marker genes. In this case the activity of the tested compound is deemed to be increased.
2. The expression level for all tested marker genes for a test compound are higher or equal than for a reference compound and the mean value of the tested compounds is outside/beyond the two-fold change area in more than half of the tested marker genes. In other words, for more than half of the tested marker genes the mean values for the expression is more distant from the graph center than the two-fold change area. In this case the activity of the tested compound is deemed to be decreased.
3. The mean values for the expression level for a test compound for more than half of the tested marker genes is within the two-fold change area of the reference compound. In this case the activity of the tested compound is deemed to be similar to the activity of the reference compound.
4. The mean values for the expression level of the marker genes of the test compound are outside the two-fold change area for more than half of the tested markers and the mean values are partially beyond the outer rim, i.e. more than two-fold, and partially are beyond the inner rim and close to the center, i.e. less than half of the values obtained for the reference compound. In other words, the mean values obtained for more than half of the tested markers are on both sides of the two-fold change area. In this case the activity of the tested compound is deemed to be incomparable with the reference compound.

A cell-based assay for assessing the effect of a test compound for inhibiting TNFα activity is herein presented. A chondrocyte cell model was set up for evaluation of the functional effect of medicines (biopharmaceuticals, small molecules) on cartilage tissue. The model can either be a 2D or a 3D model. The methods were optimised and the cultivation/testing system was miniaturised. It was confirmed that the cell model is an efficient testing system for evaluation of the anti-TNFα activity.

In one embodiment, the assay utilizes as a testing platform a primary human chondrocyte two-dimensional cell culture. Upon TNFα treatment, the cells start to express a set of marker genes. Suitable marker genes are presented in Table 4 and marked with an asterisk. The set of marker genes comprises at least 6, or at least 8, or at least 10 genes, which are presented in Table 4.

Decreased gene expression in the presence of test compound indicates that the compound has TNFα inhibitory activity. The degree of inhibition is evaluated by comparison to known anti-TNFα biological drugs (infliximab and etanercept). The system allows discrimination between the tested compounds. The preferred method for assessing gene expression is RT-qPCR.

An assay based on cartilage-derived cells, a primary diseased tissue in RA, enables a better functional characterization of new anti-TNFα therapeutics *in vitro.* The testing system of the present invention provides evidence for more efficient and reliable lead selection during pre-clinical development.

The compounds identified, characterised, and/or evaluated by the methods of the present invention can be used as agents in treating or preventing diseases associated with elevated TNFα levels, such as inflammatory diseases, such as RA, OA, and other cartilage diseases.

### References

(1) http://www.fda.gov/NewsEvents/Newsroom/PressAnnouncements/ucm291232 .htm
(2) http://www.fda.gov/downloads/Drugs/GuidanceComplianceRequlatoryInformation/Guidances/UCM291134.pdf)
(3) Tak PP, Bresnihan B. (2000): The pathogenesis and prevention of joint damage in rheumatoid arthritis: advances from synovial biopsy and tissue analysis. Arthritis Rheum 43(12): 2619-33.
(4) Kukar M, Petryna O, Efthimiou P. (2009): Biological targets in the treatment of rheumatoid arthritis: a comprehensive review of current and in-development biological disease modifying anti-rheumatic drugs. Biologics 3: 443-57.
(5) Rasheed Z, Haqqi TM. (2008): Update on Targets of Biologic Therapies for Rheumatoid Arthritis. Curr Rheumatol Rev 4(4): 246.
(6) Nesbitt A, Fossati G, Bergin M, Stephens P, Stephens S, Foulkes R, Brown D, Robinson M, Bourne T. (2007) Mechanism of action of certolizumab pegol (CDP870): in vitro comparison with other anti-tumor necrosis factor alpha agents. Inflamm Bowel Dis 13(11): 1323-32.
(7) Chiu WC, Lai YP, Chou MY. (2011) Humanization and characterization of an anti-human TNF-α murine monoclonal antibody. PLoS One 6(1):e16373.
(8) Gay RD, Clarke AW, Elgundi Z, Domagala T, Simpson RJ, Le NB, Doyle AG, Jennings PA. (2010) Anti-TNFα domain antibody construct CEP-37247: Full antibody functionality at half the size. Mabs 2(6): 625-38.
(9) Shealy D, Cai A, Staquet K, Baker A, Lacy ER, Johns L, Vafa O, Gunn G 3rd, Tam S, Sague S, Wang D, Brigham-Burke M, Dalmonte P, Emmell E, Pikounis B, Bugelski PJ, Zhou H, Scallon B, Giles-Komar J. (2010) Characterization of golimumab, a human monoclonal antibody specific for human tumor necrosis factor alpha. Mabs 2(4). [Epub ahead of print]
(10) ABI PRISM® 7700 Sequence Detection System User Bulletin #2

### Examples

Preferred embodiments of the invention are outlined in the following examples which should not be interpreted as restricting the scope or spirit of the invention.

### Example 1: Chondrocyte isolation and culture

Cartilage samples from human femoral condyles (6 specimens) were obtained from the surplus material of patients scheduled for the ACI procedure or procured postmortem from healthy donors in the first 24 h after a sudden traumatic death.

Cartilage samples were rinsed with phosphate-buffered saline (PBS), diced into small pieces, and collected in DMEM/F-12 medium supplemented with 50 mg/ml gentamycin, 2 mg/ml fungizone, 1 mg/ml collagenase type II (all produced by Gibco). The samples were digested for 24 h at 37°C. After washing and resuspending the cells in DMEM/F-12 medium, their number and viability were assessed by the exclusion of 0.2% Trypan blue (Gibco) using a hemocytometer. Chondrocytes plated in tissue culture flasks at a density of 3000 cells/cm² were cultured in a humidified incubator at 37°C and 5% CO₂. The growth medium for all cultures [DMEM/F-12, 10% (v/v) FBS (Gibco), and 50 mg/ml gentamycin] was changed biweekly. Cells were cultivated until 80% of confluency was achieved. Cells were detached by the trypsin-EDTA solution (Sigma). To investigate the effect of TNFα and anti-TNFα biologics, chondrocytes of the second (P2) or third (P3) passage were used.

### Example 2: Stimulation of chondrocytes with TNFα and anti-TNFα biologics

To assess the effectiveness of infliximab and etanercept on chondrocyte gene expression, four different samples of one primary culture cells were prepared:
- non-stimulated chondrocytes
- chondrocytes stimulated with TNFα
- chondrocytes stimulated with TNFα + infliximab
- chondrocytes stimulated with TNFα + etanercept

P2 or P3 chondrocytes were cultured until 100% confluence was achieved. After 24 hour incubation in a serum-free medium, cells were stimulated by addition of 1 ng/ml recombinant human TNFα (Peprotech). For studying the neutralization effect of infliximab and etanercept, different concentrations of each drug were preincubated with TNFα for 30 min, and afterwards added to chondrocyte culture. For qPCR array and antibody-based array experiments, 1µg/ml of anti-TNFα biologics was used. After 24 hours, chondrocytes were sampled for mRNA analysis and conditioned media for protein assessment.

### TNFα and anti-TNFα stock preparation

TNFα (Peprotech) was prepared according to the manufacturer's protocol. 10 µg/ml stocks were aliquoted and kept at -20°C.

Infliximab (Remicade®, Centocor) - 100 mg of lyophilized powder was reconstituted with 10 ml of sterile water to obtain a stock solution of 10 mg/ml.

Etanercept (Enbrel®, Wyeth Pharmaceuticals) - 25 mg of etanercept was reconstituted with 1 ml of sterile water to obtain a stock solution of 25 mg/ml.

Further dilutions of both anti-TNFα agents were prepared with PBS or DMEM/F-12 medium. Stock solutions were aliquoted and stored at -20 or -80°C.

### RT-qPCR

### RNA isolation

Total RNA was isolated using GenElute™ Mammalian Total RNA Miniprep Kit (Sigma) according to the manufacturer's protocol. The amount of RNA was quantified by RiboGreen fluorescent assay (Molecular Probes) according to the manufacturer's protocol. The integrity of denaturated RNA samples was checked on FlashGel™ System (Lonza).

### Reverse transcription

From the total RNA (usually 0.5 - 1 µg) cDNA was generated using High-Capacity cDNA Reverse transcription Kit (Applied Biosystems) according to the instructions.

Kit contains random hexamer primers that enable transcription of the first strand mRNA with the same efficiency for all amplicons in the sample, as this is one of the basic requirements for gene expression quantification analysis. Until use, cDNA was stored at -20°C.

### qPCR

qPCR was performed in duplicates or triplicates in 384-well optical plate (10 µl reactions) using ABI 7900HT Sequence Detection System or *7900HT* Fast Real-Time PCR System (Applied Biosystems). TaqMan® gene expression master mix and TaqMan® gene expression assays were used (Applied Biosystems). Assays were selected not to detect genomic DNA. Assays ID, gene name and gene symbol are presented in Table 1. After validation of four endogenous control genes, GUS was chosen because its expression was constant in all experimental conditions. Data analysis was performed using ABI 7900HT Sequence Detection System version 2.4 and RQ Manager software (Applied Biosystems). For each cDNA sample, the Cq value was determined as the cycle number at which the fluorescence intensity reached the threshold 0.1. The C_{q} value of the housekeeping gene encoding GUS was subtracted to the Cq value of the target gene, to derive ΔC_{q}. The level of expression of each gene was then calculated as 2^{abs(ΔCt)}. Relative expression values indicate if the gene of interest is expressed to a greater or lower extent than the endogenous control gene. Relative expression values are reported in arbitrary units and can only be used for comparative analysis among samples. For qPCR array analysis 2^{-ΔΔCt} formula was applied. Data was normalized to non-treated samples. Results are presented as Log₂ relative quantity. For dose-response assessment, data was normalized to TNFα treated samples (10).

From the studies using qPCR array, good-responsive genes were identified. Genes were chosen according to their role in chondrocyte extracellular matrix remodeling, inflammatory response, signal transduction pathways, etc. In Table 1 array position, gene symbol, gene name and assay ID (Applied Biosystem) are presented.

**Table 1: Genes analyzed in qPCR experiments**

| Array position | Gene Symbol | Gene Name | Assay ID |
|---|---|---|---|
| 1 | ADAM17 | ADAM metallopeptidase domain 17 | Hs01041915_m1 |
| 2 | ADAMTS4 | ADAM metallopeptidase with thrombospondin type 1 motif, 4 | Hs00192708_m1 |
| 3 | ADAMTS5 | ADAM metallopeptidase with thrombospondin type 1 motif, 5 | Hs00199841_m1 |
| 4 | CD44 | CD44 molecule (Indian blood group) | Hs01075861_m1 |
| 5 | COMP | cartilage oligomeric matrix protein | Hs00164359_m1 |
| 6 | PTGS2 | prostaglandin-endoperoxide synthase 2 (prostaglandin G/H synthase and cyclooxygenase) | Hs00153133_m1 |
| 7 | HAPLN1 | hyaluronan and proteoglycan link protein 1 | Hs00157103_m1 |
| 8 | CHUK | conserved helix-loop-helix ubiquitous kinase | Hs00175141_m1 |
| 9 | IL1B | interleukin 1, beta | Hs01555410_m1 |
| 10 | IL1RA | interleukin 1 receptor antagonist | Hs00893626_m1 |
| 11 | IL32 | interleukin 32 | Hs00170403_m1 |
| 12 | IL6 | interleukin 6 (interferon, beta 2) | Hs00985639_m1 |
| 13 | IL8 | interleukin 8 | Hs00174103_m1 |
| 14 | MAPK1 | mitogen-activated protein kinase 1 | Hs01046830_m1 |
| 15 | MCP1 | chemokine (C-C motif) ligand 2 | Hs00234140_m1 |
| 16 | CCL13 | chemokine (C-C motif) ligand 13 | Hs00234646_m1 |
| 17 | MAP2K3 | mitogen-activated protein kinase kinase 3 | Hs00177127_m1 |
| 18 | MMP1 | matrix metallopeptidase 1 (interstitial collagenase) | Hs00899658_m1 |
| 19 | MMP10 | matrix metallopeptidase 10 (stromelysin 2) | Hs00233987_m1 |
| 20 | MMP13 | matrix metallopeptidase 13 (collagenase 3) | Hs00233992_m1 |
| 21 | MMP3 | matrix metallopeptidase 3 (stromelysin 1, progelatinase) | Hs00968305_m1 |
| 22 | NOS2 | nitric oxide synthase 2, inducible | Hs01075529_m1 |
| 23 | NFKB1 | nuclear factor of kappa light polypeptide gene enhancer in B-cells 1 | Hs00765730_m1 |
| 24 | RUNX2 | runt-related transcription factor 2 | Hs00231692_m1 |
| 25 | SOX9 | SRY (sex determining region Y)-box 9 | Hs00165814_m1 |
| 26 | TIMP1 | TIMP metallopeptidase inhibitor 1 | Hs00171558_m1 |
| 27 | TIMP2 | TIMP metallopeptidase inhibitor 2 | Hs00234278 m1 |
| 28 | TLR1 | toll-like receptor 1 | Hs00413978 m1 |
| 29 | TLR2 | toll-like receptor 2 | Hs00610101_m1 |
| 30 | TLR4 | toll-like receptor 4 | Hs01060206_m1 |
| 31 | TNF | tumor necrosis factor | Hs00174128 m1 |
| 32 | TNFRSF1A | tumor necrosis factor receptor superfamily, member 1A | Hs00533560 m1 |
| 33 | TNFRSF1B | tumor necrosis factor receptor superfamily, member 1B | Hs00153550_m1 |
| 34 | VCAM1 | vascular cell adhesion molecule 1 | Hs01003372_m1 |
| 35 | VEGFA | vascular endothelial growth factor A | Hs00900055_m1 |
| 36 | ACAN | aggrecan | Hs00153936_m1 |
| 37 | BGN | biglycan | Hs00959143_m1 |
| 38 | COL11A2 | collagen, type XI, alpha 2 | Hs00365416_m1 |
| 39 | COL12A1 | collagen, type XII, alpha 1 | Hs00189184_m1 |
| 40 | COL9A3 | collagen, type IX, alpha 3 | Hs00951243_m1 |
| 41 | DCN | decorin | Hs00266491_m1 |

### Example 3: Antibody-based array

A custom antibody-based array (RayBiotech) designed to detect 10 different proteins including cytokines, matrix metalloproteinase, chemokine, etc. (Figure 2) was used.

Experiments were performed essentially as recommended by the manufacturer. Briefly, array glass slide containing 14 subarrays was incubated for 30 min in blocking buffer, then incubated overnight with 100 µl of conditioned media at 4°C and washed. A cocktail of biotin-conjugated antibodies was added and incubated for 2 hours at room temperature. After washing, fluorescent dye-conjugated streptavidin was added to each subarray and incubated for 2 hours at room temperature protected from light. After extensive washing and drying, the signal was obtained using cy3 channel (excitation frequency = 532 nm) on GenePix Personal 4100A microarray scanner. As a negative control of the array, DMEM/F-12 was used. Spots of the array were quantified by the image analysis GenePix® Pro 6 software in duplicates. Log₂ transformation of signal intensities was performed. Three different biological samples with TNFα and TNFα + anti-TNFα biologics were analyzed and samples were normalized to non-treated controls.

### Example 4: Optimization of TNFα concentration

Optimization of TNFα concentration was performed on 3 biological samples. Results of testing 3 different TNFα concentrations (1, 10 and 50 ng/ml) in one representative biological sample are shown in Table 2.

**Table 2: Gene expression vs.TNFα concentration.**

| | **MMP1** | **TLR2** | **COX2** | **IL-6** |
|---|---|---|---|---|
| **DS TNF = 0** | 17.30 | 3.68 | 21.50 | 13.22 |
| **DS TNF = 1 ng/ml** | 1607.07 | 295.14 | 162.47 | 1626.94 |
| **DS TNF = 10 ng/ml** | 2464.36 | 454.32 | 498.46 | 2340.04 |
| **DS TNF = 50 ng/ml** | 2733.06 | 336.77 | 731.17 | 1789.96 |

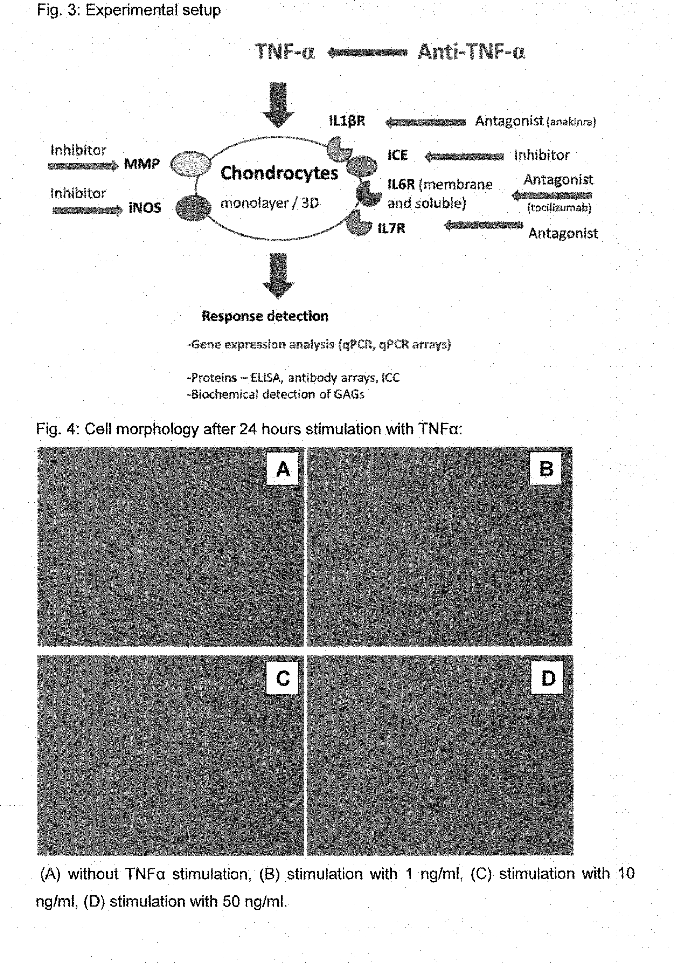

According to the obtained results, TNFα concentration of 1 ng/ml was chosen for all further experiments. At 1 ng/ml, the differences in gene expression among stimulated and non-stimulated samples are sufficiently large. Cell morphology and viability was not affected at any of the tested concentrations (Figure 4).

These experiments served also for the selection of good-response genes. Considering the results, genes shown in Table 2 MMP1, TLR2, COX2 and IL-6, were selected for further experiments.

### Example 5: Assessing the effect of infliximab on gene expression

In this preliminary study, the effect of 10 and 100 µg/ml of infliximab on gene expression were assessed. Cells were stimulated with 10 ng/ml of TNFα. The results are presented in the Table 3. It is seen that the expression of some genes (MMP1 and IL-6) is affected by increasing infliximab concentration. This gave the inventors the idea to try to determine IC₅₀ values for the expression of different genes. IC₅₀ would be the concentration of anti-TNFα biologics at which 50% gene expression, compared to TNFα stimulated cells, would be achieved. Theoretically, the highest anti-TNFα concentration should block the expression completely (to the same level as in a non-stimulated sample), while the lowest concentration shouldn't block gene expression at all (the same expression level as in a TNFα stimulated sample).

**Table 3: Gene expression vs.different infliximab concentrations.**

| | | **MMP1** | **TLR2** | **COX2** | **IL-6** |
|---|---|---|---|---|---|
| **PO TNF = 0** | | 3.20 | 4.58 | 23.40 | 17.63 |
| **PO TNF = 10** | | 10756.00 | 1816.10 | 892.98 | 2920.51 |
| **PO TNF = 10, infliximab = 10** | | 501.23 | 127.46 | 280.80 | 560.06 |
| **PO TNF = 10, infliximab = 100** | | 345.87 | 134.02 | 292.94 | 457.05 |

qPCR array studies

qPCR array was designed to assess gene expression of genes involved in extracellular matrix synthesis or degradation, inflammatory response, stress response and involvement in signaling/regulatory pathways (Table 1).

### TNFα stimulation and anti- TNFα addition

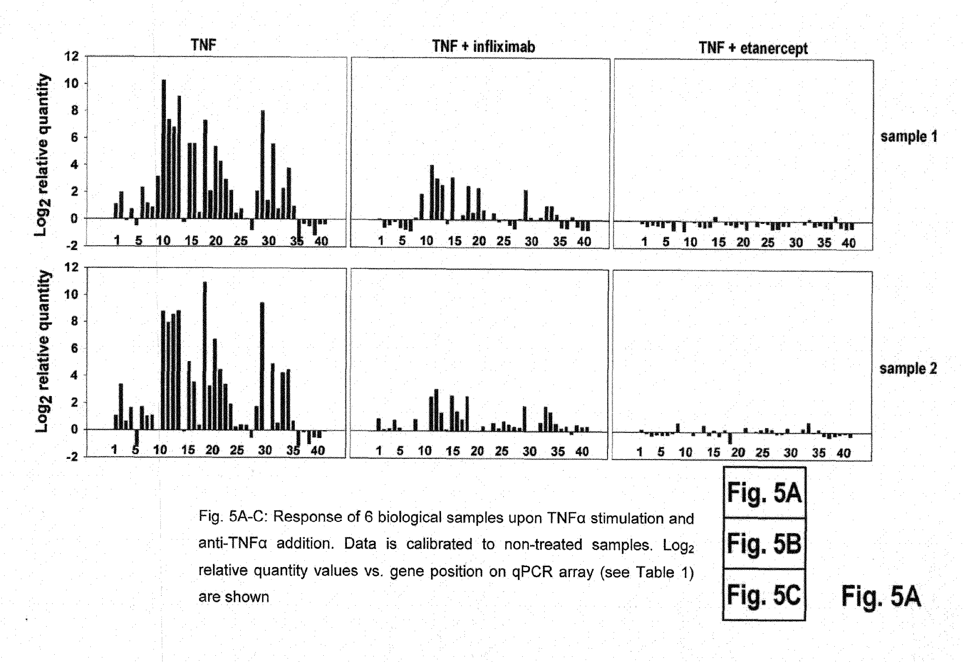

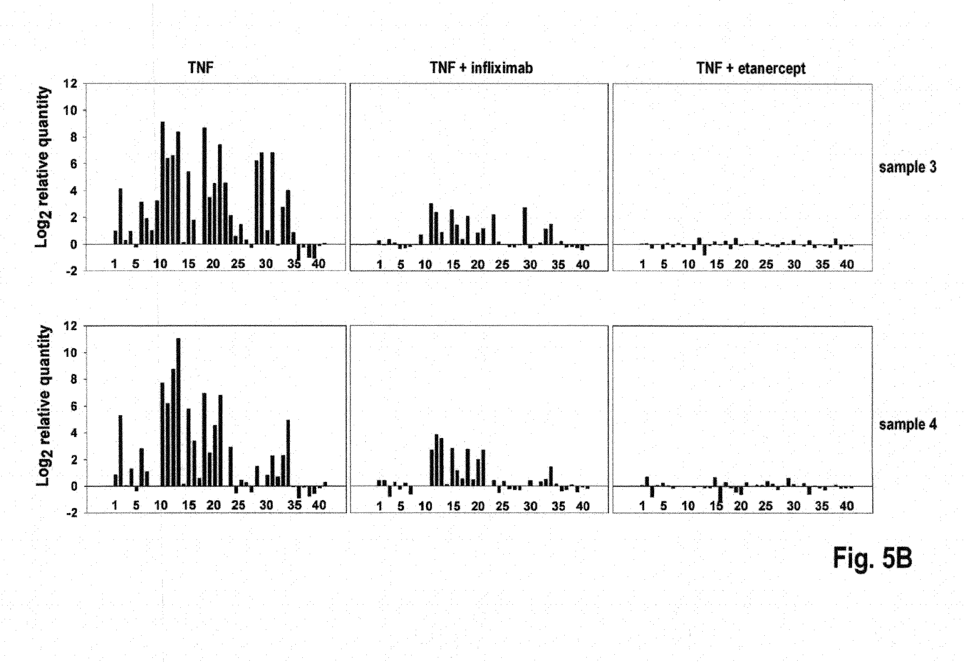

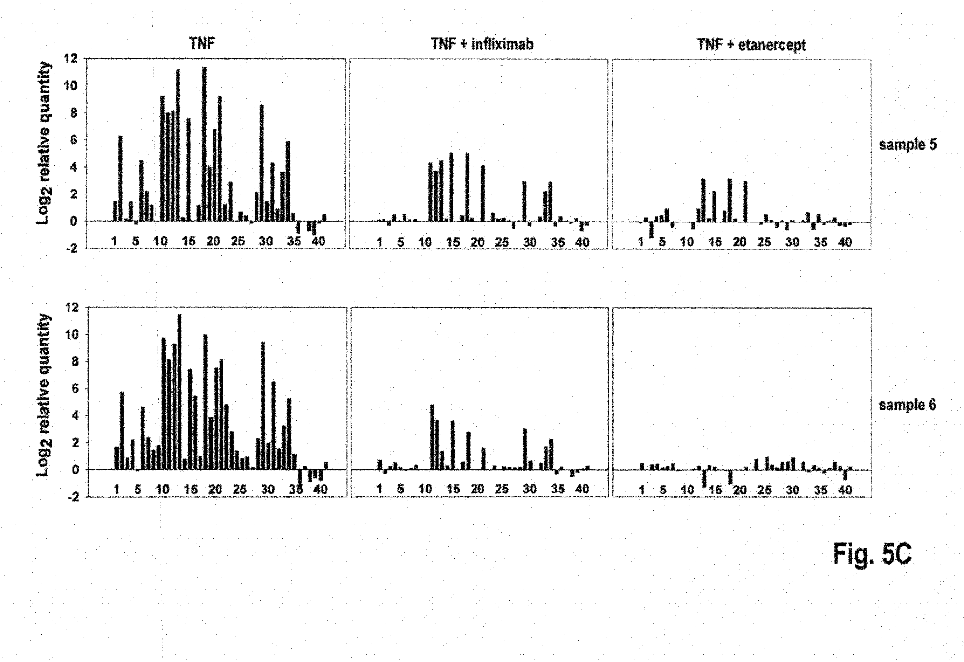

Figure 5 shows response of different biological samples upon TNFα stimulation and addition of anti- TNFα biologics. In the graph Log₂ relative quantity vs. particular gene expression is presented. Gene number and position is seen from Table 1. Calibrator is a non-treated sample. Higher column means higher gene expression upon TNFα stimulation. On the other hand, value 0 mean total inhibition of TNFα by anti- TNFα biologics.

### TNFα stimulation:

Results show similar expression profiles in different biological samples. Approximately same sets of genes are expressed in all samples i.e. strongly upregulated are ADAMTS-4, set of different interleukins (IL-32, IL-6, IL-8), chemokine MCP-1, matrix metalloproteinases, TLR2, TNFRSF1B, VCAM-1. Slightly downregulated are genes involved in matrix formation e.g. aggrecan and different collagens. Relative quantity (RQ) values are presented in Table 4.

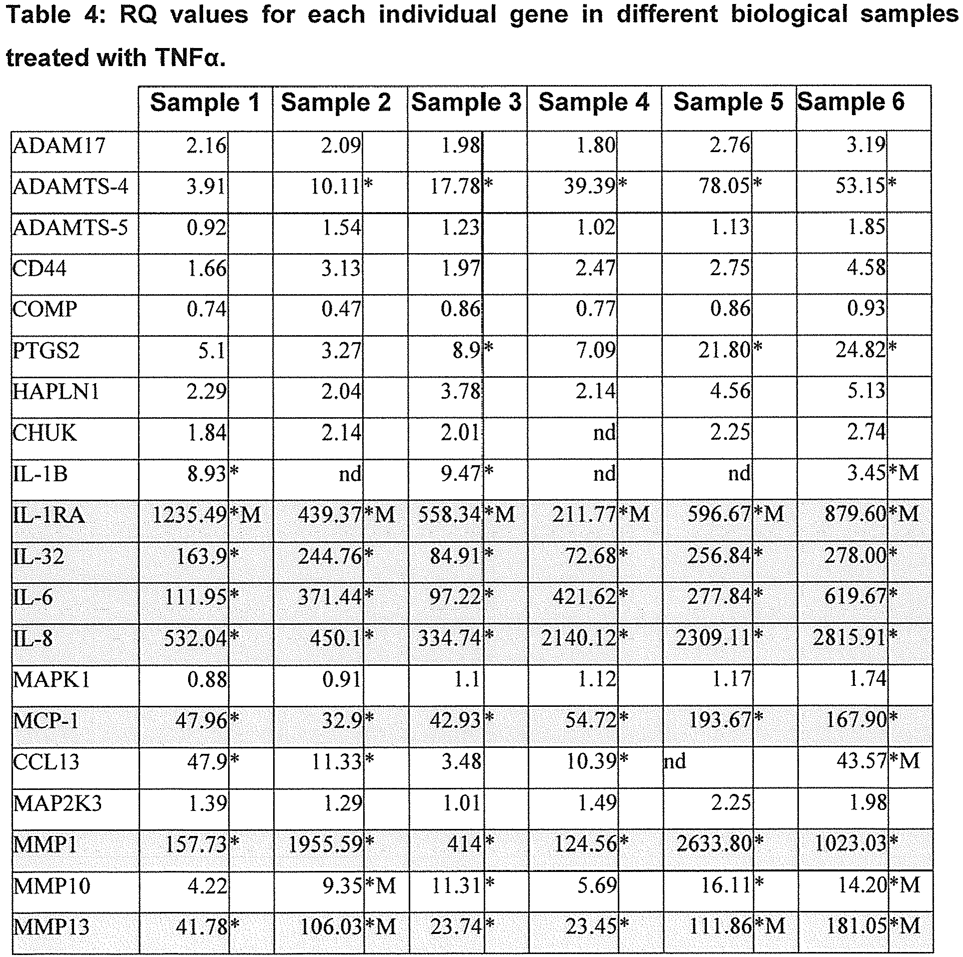

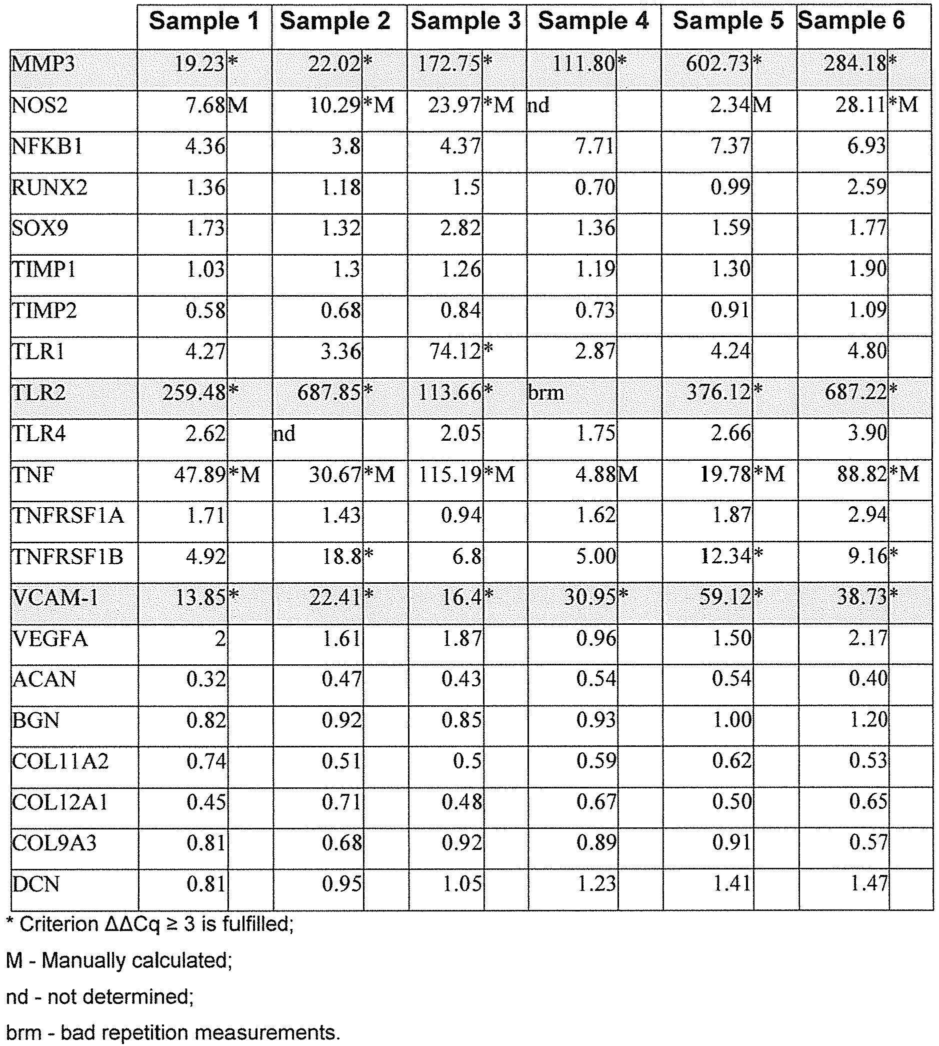

A criterion for good-response gene was set at: ΔΔCq ≥ 3. This means that the differences between non-stimulated and TNFα stimulated samples are big enough for detection of differences in gene suppression upon anti-TNFα biologics addition.

The expression of some genes in non-stimulated conditions is below detection limit, but after the stimulation, expression increases importantly. For these genes, the RQ value was calculated using the approximation of Cq = 35 for non-stimulated samples. These measurements are labeled with "M".

Genes marked with grey color are selected as "the most representative" genes. They fulfilled the ΔΔCq ≥ 3 criterion in all 6 examined biological samples. These genes are considered as marker genes for TNFα-induced chondrocyte expression.

### Addition of anti-TNFα biologics:

Two clinically approved anti-TNFα drugs were tested: infliximab and etanercept. Neutralization capacity of both anti-TNFα biologics led to downregulation of determined genes. Among different biological samples, variability in degree of downregulation was observed.

Figure 5 shows also downregulation of genes in different biological samples upon infliximab and etanercept treatment. Results present neutralization efficiencies of anti-TNFα biologics compared to initial gene expression levels. From these graphs it can be seen if the effect of TNFα can be blocked completely (values around 0).

From the results it is obvious that etanercept blocks TNFα induced gene expression better than infliximab. Except in sample 5, etanercept almost completely abolished gene expressions. In sample 5, etanercept could not abolish the expression of IL-8, MCP-1, MMP1 and MMP3. Genes whose expression was not abolished by infliximab in most samples were: IL-32, IL-6, IL-8, MCP-1, MMP1, MMP3, MMP13, TLR2, TNFRSF1B and VCAM-1.

However, genes that are mostly suppressed by both anti-TNFα biologics are: ADAMTS4, PTGS2, interleukins (to highest extent IL-8), chemokines (MCP-1 and CCL13), matrix metalloproteinases (to highest extent MMP1 and MMP3), TLR2 and VCAM-1.

### Example 6: Dose-response assessment

Dose-response assessment was performed in 6 biological samples. The widest tested concentration range of infliximab and etanercept was from 1000 to 10⁻⁴ µg/ml.

In Table 5 gene expression values obtained from one representative biological sample are shown.

**Table 5: Gene expression values vs. different anti-TNFα biologics concentration**

| | **IL-1 RA** | **IL-6** | **IL-8** | **MMP1** | **MMP3** | **TLR2** | **TNF** | **VCAM-1** |
|---|---|---|---|---|---|---|---|---|
| DG 0 | nd | 1.09 | 0.24 | 1.31 | 3.24 | 1.01 | nd | 202.97 |
| DG TNF | 66.49 | 269.90 | 879.54 | 2175.51 | 1917.05 | 210.18 | 2.99 | 5926.64 |
| DG I 1000 | 0.34 | 2.95 | 5.80 | 17.12 | 15.58 | 3.68 | nd | 461.29 |
| DG I 100 | nd | 3.29 | 2.12 | 7.75 | 21.86 | 2.71 | nd | 607.16 |
| DG I 10 | nd | 6.82 | 3.53 | 18.81 | 34.43 | 4.52 | nd | 1150.86 |
| DG I 1 | nd | 10.11 | 7.53 | 23.84 | 54.02 | 7.64 | nd | 1216.53 |
| DG I 0.1 | nd | 20.26 | 13.48 | 31.78 | 85.54 | 9.44 | nd | 1889.73 |
| DG I 0.01 | 0.28 | 102.14 | 111.29 | 244.51 | 742.09 | 67.45 | nd | 4186.14 |
| DG I 10^-3 | 21.37 | 259.87 | 635.33 | 2272.13 | 3201.71 | 219.28 | 0.69 | 7995.24 |
| DG I 10^-4 | 51.69 | 461.76 | 1322.78 | 3239.95 | 4281.42 | 447.02 | 1.89 | 13601.81 |

| | **IL-1RA** | **IL-6** | **IL-8** | **MMP1** | **MMP3** | **TLR2** | **TNF** | **VCAM-1** |
|---|---|---|---|---|---|---|---|---|
| DG 0 | nd | 1.09 | 0.24 | 1.31 | 3.24 | 1.01 | nd | 202.97 |
| DG TNF | 66.49 | 269.90 | 879.54 | 2175.51 | 1917.05 | 210.18 | 2.99 | 5926.64 |
| DG E 1000 | nd | 2.83 | 6.11 | 26.04 | 45.61 | 1.45 | nd | 190.59 |
| DG E 100 | nd | 2.15 | 4.49 | 9.06 | 36.85 | 1.07 | nd | 185.87 |
| DG E 10 | nd | 1.69 | 2.80 | 6.32 | 26.71 | 0.68 | nd | 159.75 |
| DG E 1 | nd | 1.85 | 2.72 | 8.86 | 29.95 | 0.84 | nd | 149.07 |
| DG E 0.1 | nd | 1.29 | 3.11 | 2.43 | 27.85 | 1.33 | nd | 227.15 |
| DG E 0.01 | nd | 4.05 | 4.38 | 6.51 | 34.79 | 3.44 | nd | 746.82 |
| DG E 10^-3 | nd | 2.73 | 2.21 | 2.97 | 14.54 | 2.52 | nd | 640.54 |
| DG E 10^-4 | 1.24 | 243.10 | 647.62 | 594.28 | 2008.10 | 91.34 | 0.09 | 5918.60 |

| | | | | | | | | |
|---|---|---|---|---|---|---|---|---|
| nd - not determined (Ct ≥ 35); I - infliximab, E - etanercept | | | | | | | | |

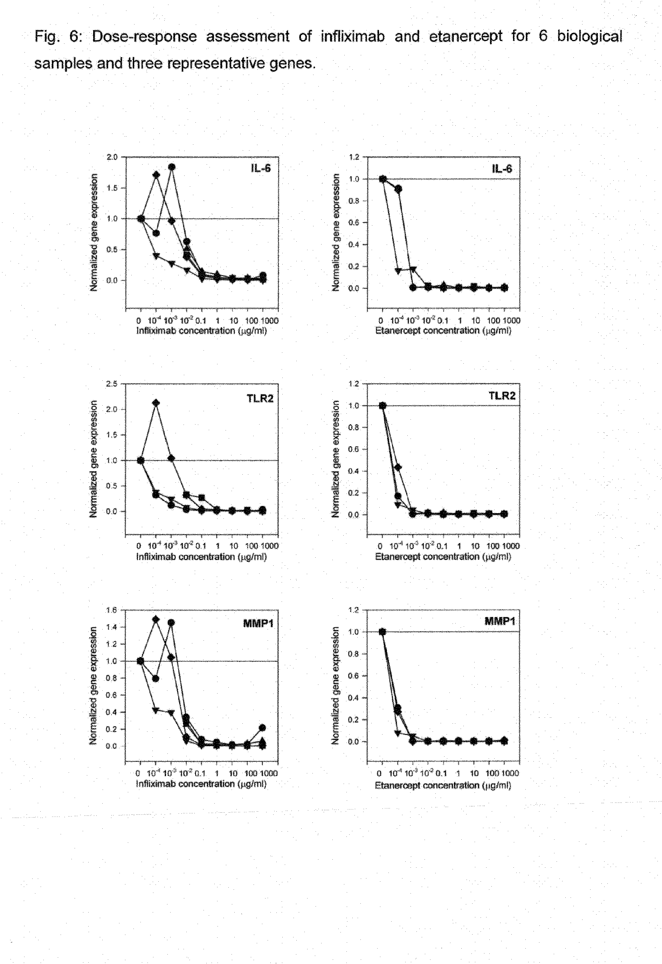

In Figure 6 dose-response assessment for infliximab and etanercept for 6 biological samples and three genes is shown. Data was normalized to samples treated with TNFα.

From these results it is seen that IC₅₀ values cannot be determined by qPCR method. In the case of infliximab it is seen that not even the highest concentrations blocked TNFα completely. Low infliximab concentrations (10^{^-3} to 10^{^-4} µg/ml) even increased gene expression in some samples.

With etanercept even less dose-dependent effect was observed. Similar neutralization effect was observed in a concentration range from 10^{^3} to 10^{^-3} µg/ml. In this concentration range etanercept abolished completely the expression of the tested genes. Etanercept at 10^{^-4} µg/ml had less effect on blocking gene expression induced by TNFα than at higher concentrations.

In conclusion, after testing 6 biological samples it seems that etanercept is more effective in blocking gene expression than infliximab in a large concentration range.

### Example 7: Assessment of assay's reproducibility

To assess if the same biological sample responds approximately in the same manner upon another TNFα and anti-TNFα treatment, qPCR array experiment with 3 biological samples was repeated more than 6 months later.

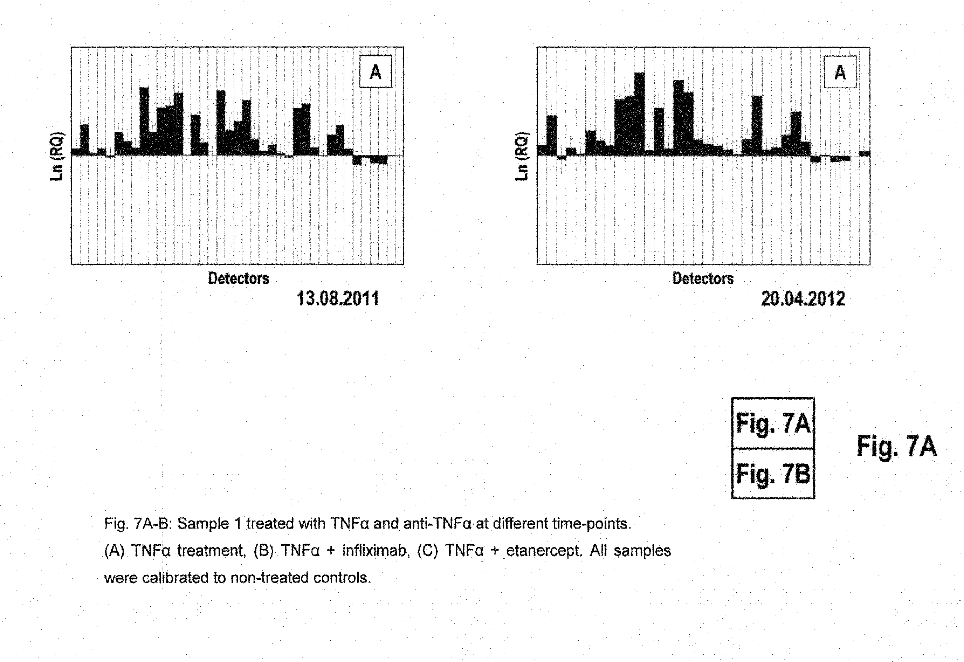

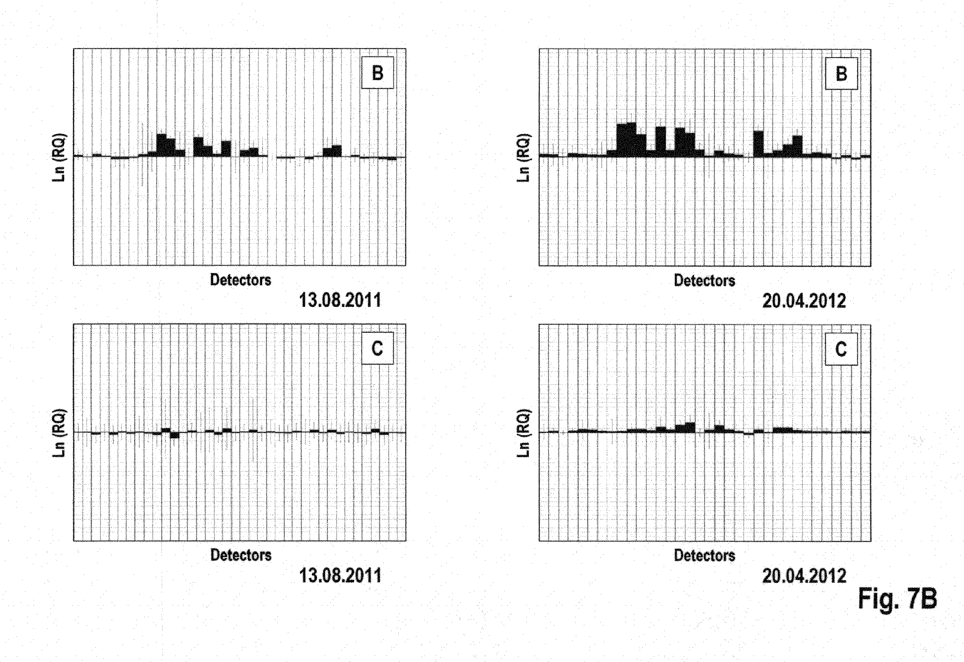

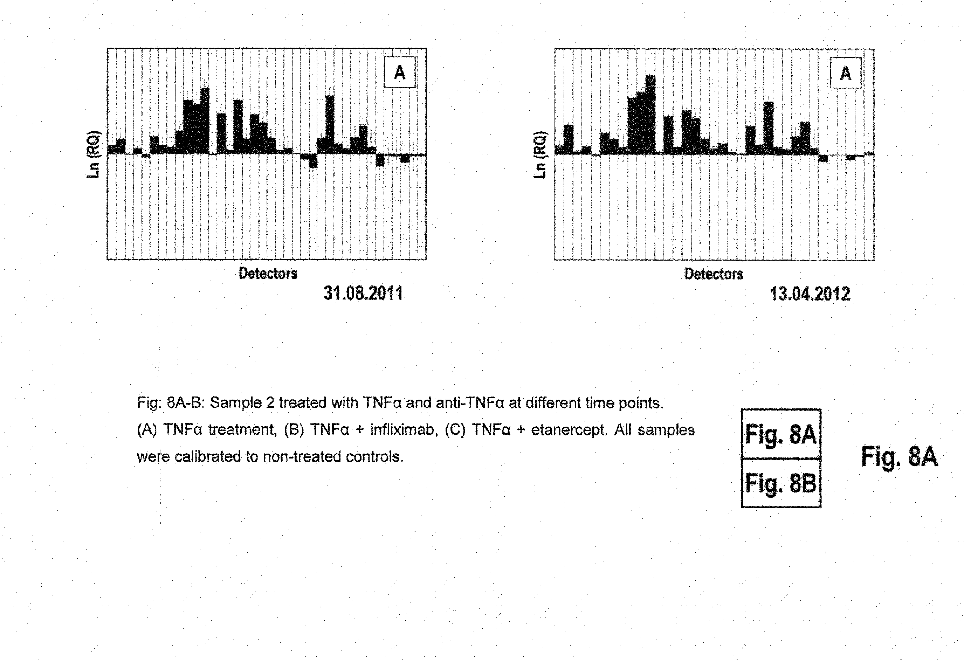

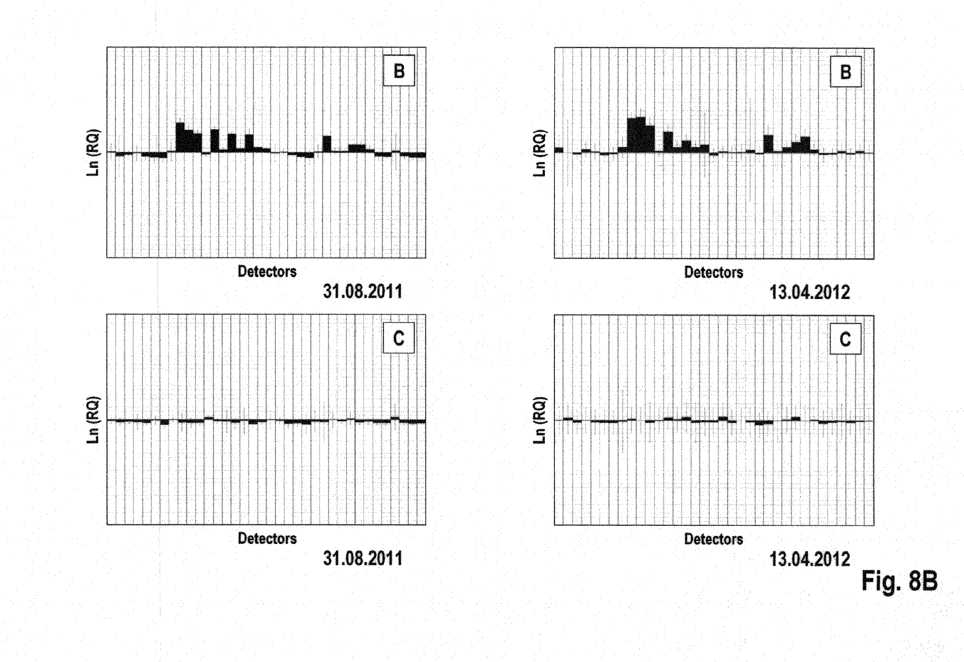

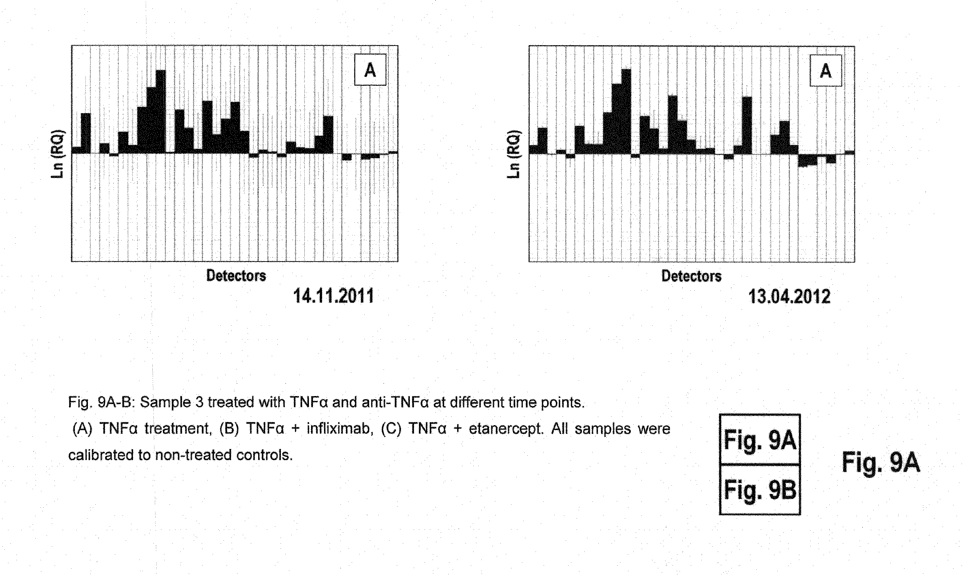

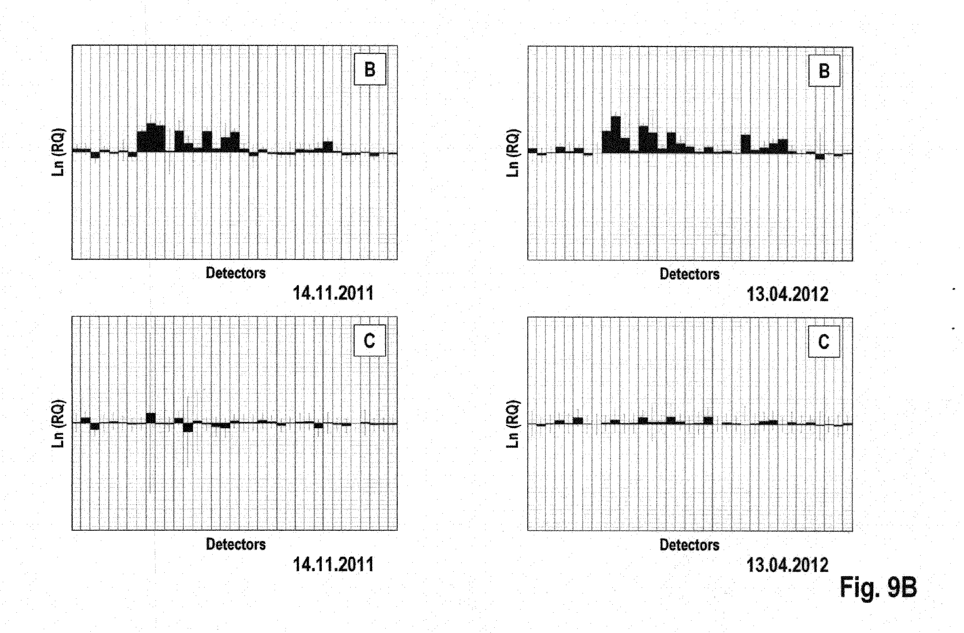

Chondrocytes of three biological samples of the same passage as in the first assay were unfrozen and seeded for the same experimental procedure. The same data analysis was performed. In Figures 7 to 9, results obtained at two different time-points are presented. In the Figures, Ln(RQ) vs. particular gene expression is presented. Figures serve just to compare gene expression profiles between both time-points. And it is evident that expression profiles upon TNFα stimulation and anti-TNFα addition are very similar at both time-points.

Response of biological repetitions upon TNFα stimulation is shown in Table 6. RQ values for each individual gene are presented. The data was analyzed and presented in the same way as it is described for Table 4.

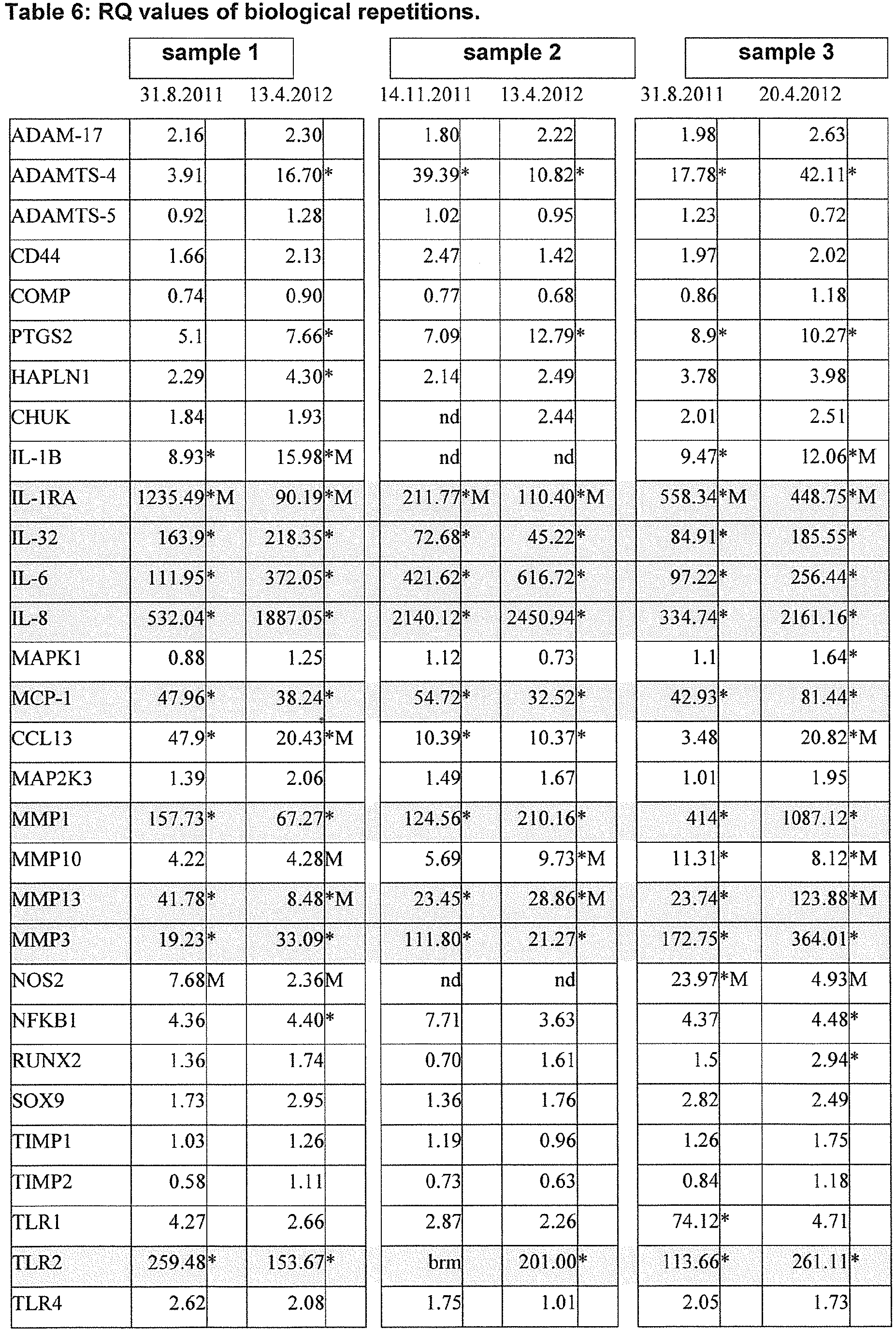

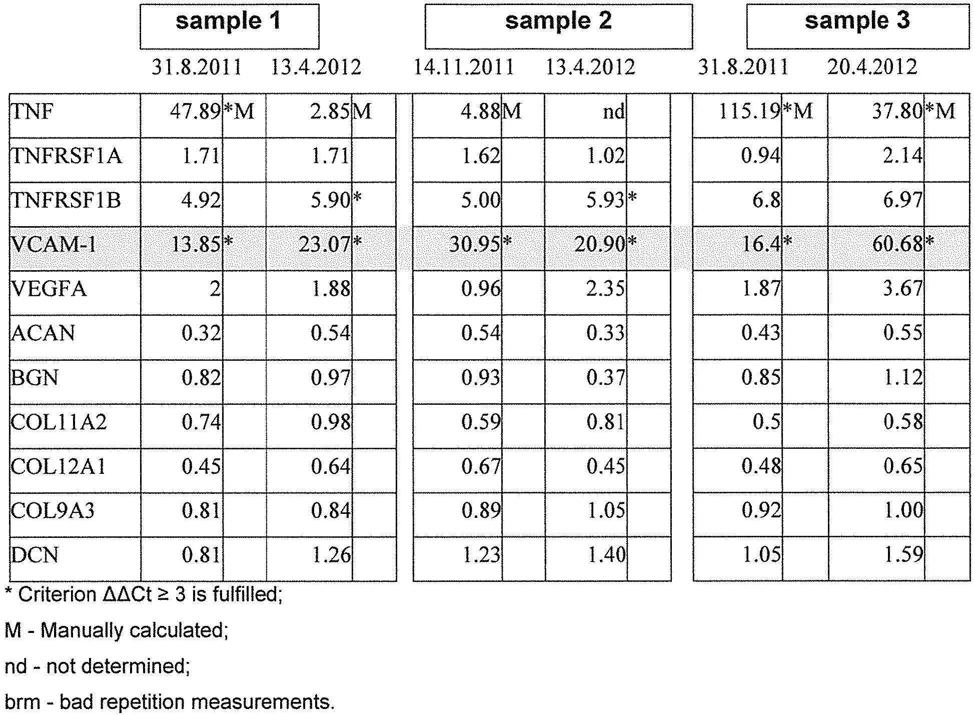

The results presented in Table 6 show that all three biological repetitions responded to TNFα stimulation in a similar manner as their counterparts. As expected, RQ values within the same biological sample differ. However, what matters is that the response of genes, corresponding to marker genes from Table 4, meet the criteria ΔΔCₜ ≥ 3 in all analyzed samples, too. The use of these marker genes seems to be suitable for functional analysis of new anti-TNFα biologics on chondrocyte cell model.

### Example 8: Antibody-based array

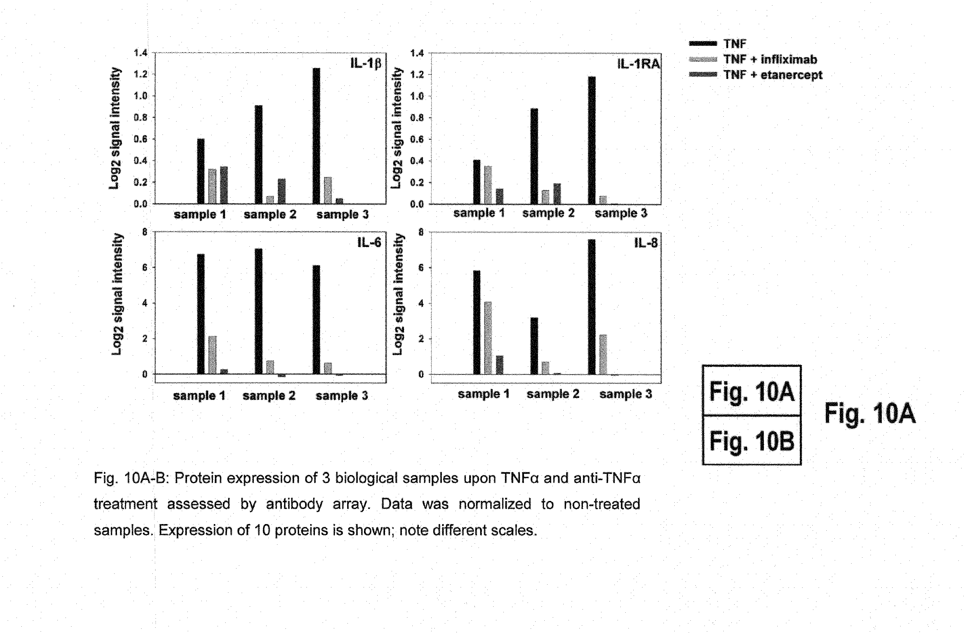

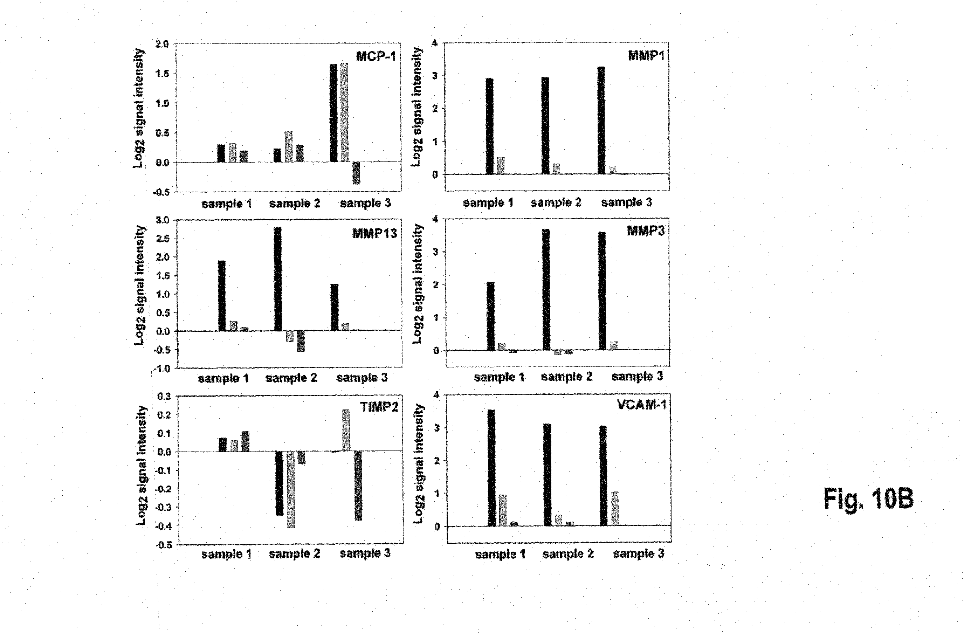

To confirm the data obtained by gene expression analysis, protein expression was assessed using custom designed antibody-based array. Proteins, whose gene expression changed importantly upon TNFα addition were chosen: IL-1ra, IL-1beta, IL-6, IL-8, MMP1, MMP3, MMP13, MCP-1, TIMP2 and VCAM-1. The results of three biological samples are presented in Figure 10.

From the results it is seen that IL-6, IL-8, MMP1, MMP3, MMP13 and VCAM-1 protein expressions also depends upon anti-TNFα treatment. Etanercept is more efficient in abolishing the expression of the above mentioned proteins than infliximab. The data obtained in gene expression level was confirmed on protein level for 6 marker genes.

## Claims

1. In vitro method for assessing TNFα inhibitory activity of a test compound, the method comprising
a) contacting a chondrocyte cell culture with TNFα in
i) absence of said test compound,
ii) absence of said test compound but in presence of a known compound having TNFα inhibitory activity, and in
iii) presence of said test compound but in absence of said known compound having TNFα inhibitory activity;
b) thereafter measuring the expression levels of a set of marker genes in each option i), ii), and iii) of step a), wherein the set of marker genes comprises at least 6 marker genes, wherein the marker genes are selected from a group comprising at least IL-1RA, IL-6, IL-8, IL-32, MMP1, MMP3, MMP13, MCP-1, TLR2, and VCAM-1; and
c) assessing the levels of expression of said set of marker genes,
wherein a decrease of the mean value of the expression level of a marker gene of the test compound in more than half of the measured expression levels of marker genes to less than half the mean value compared with the expression level of the corresponding marker gene of the known reference compound indicates that the test compound has an increased TNFα inhibitory activity compared to the known compound; and wherein an increase of the mean value of the expression level of a marker gene of the test compound in more than half of the measured expression levels of marker genes to more than half the mean value compared with the expression level of the corresponding marker gene of the known reference compound indicates that the test compound has a decreased TNFα inhibitory activity compared to the known compound; and wherein a change of the mean value of the expression levels of more than half of the measured marker genes of the test compound of less than twice the mean value but more than half the mean value of the corresponding expression levels of the marker genes of the known compound indicates that the test compound has a similar TNFα inhibitory activity compared to the known compound.

2. Method of claim 1 for identifying TNFα inhibitory activity of the test compound, wherein decrease of mean value of the levels of expression of marker genes in presence of the test compound but in absence of said known compound having TNFα inhibitory activity compared to the corresponding levels of expression of marker genes in absence of said test compound indicates that the test compound has TNFα inhibitory activity.

3. Method of any one of the preceding claims, wherein the set of marker genes comprises at least IL-6, IL-8, MMP1, MMP3, MMP13, and VCAM-1.

4. Method of any one of the preceding claims, wherein the set of marker genes comprises at least eight marker genes.

5. Method of claim 4, wherein the set of marker genes comprises at least IL-6, IL-8, IL-32, MMP1, MMP3, MMP13, TLR2 and VCAM-1.

6. Method of any one of the preceding claims, wherein the set of marker genes comprises at least ten marker genes.

7. Method of claim 6, wherein the set of marker genes comprises at least IL-1RA, IL-6, IL-8, IL-32, MMP1, MMP3, MMP13, MCP-1, TLR2 and VCAM-1.

8. Method of any one of the preceding claims, wherein the chondrocyte cell culture is a 2D cell model, or a 3D cell model, and/or wherein the chondrocyte cell culture is a primary chondrocyte cell culture, and/or wherein the chondrocyte cell culture is a human chondrocyte cell culture.

9. Method of any one of the preceding claims, wherein the gene expression level is determined by RT-qPCR.

10. Method of any one of the preceding claims, wherein the contact time of the test compound and the known compound with the chondrocyte cell culture is at least 24 hours, before levels of expression are determined.

11. Method of any one of the preceding claims, wherein the concentration of the test compound and the known compound in ii) and iii) is substantially the same.

12. Kit for use in the method of any one of the preceding claims, wherein the kit comprises means for measuring the expression levels of a set of marker genes, wherein the set of marker genes comprises at least 6 marker genes, wherein the marker genes are selected from a group comprising at least IL-1RA, IL-6, IL-8, IL-32, MMP1, MMP3, MMP13, MCP-1, TLR2, and VCAM-1, and optionally TNFα, and optionally the known compound; wherein preferably the measuring is performed by RT-qPCR.

13. Compound having TNFα inhibitory activity identified and/or assessed by the method of any of the preceding claims for use in treating or preventing diseases associated with elevated TNFα levels, such as inflammatory diseases, such as rheumatoid arthritis (RA) or osteoarthritis (OA).
